Europäisches Patentamt

European Patent Office

Office européen des brevets

(19)

(11) Numéro de publication : **0 288 342 B1**

(12)

# FASCICULE DE BREVET EUROPEEN

(45) Date de publication du fascicule du brevet :
06.11.91 Bulletin 91/45

(51) Int. Cl.⁵ : **A61K 7/06,** A61K 7/48,
A61K 31/20, A61K 31/44,
A61K 33/30

(21) Numéro de dépôt : 88400697.4

(22) Date de dépôt : 23.03.88

(54) **Compositions cosmétiques et pharmaceutiques à application topique contenant un acide dicarboxylique de C 7 à C 13, un sel soluble de zinc et de la vitamine B6.**

(30) Priorité : 27.03.87 FR 8704332

(43) Date de publication de la demande :
26.10.88 Bulletin 88/43

(45) Mention de la délivrance du brevet :
06.11.91 Bulletin 91/45

(84) Etats contractants désignés :
BE CH DE ES FR GB GR IT LI LU NL

(56) Documents cités :
EP-A- 0 048 473
EP-A- 0 155 344
WO-A-86/06586
AU-A- 86 381
US-A- 4 386 104

(73) Titulaire : SOCIETE BFB
7, rue Michelet
F-93360 NEUILLY PLAISANCE (FR)

(72) Inventeur : Bigou, Alphonse
Z.A. La Fontaine Du Vaisseau 7 rue Michelet
F-93360 Neuilly Plaisance (FR)

(74) Mandataire : Combe, André et al
CABINET BEAU DE LOMENIE 55, rue
d'Amsterdam
F-75008 Paris (FR)

## Description

La présente invention concerne des compositions cosmétiques et pharmaceutiques à application topique contenant un acide dicarboxylique an $C_7$ à $C_{13}$, un sel soluble de zinc et de la vitamine $B_6$.

On sait que l'équipement enzymatique en 5-$\alpha$ réductase, ou l'activité de cette enzyme au niveau de la peau peuvent être excessifs et entraîner des désordres dont les principaux sont, au niveau du cuir chevelu, l'alopécie androgénogénétique et, au niveau de la peau, l'hyperséborrhée et l'acné.

On sait que les acides dicarboxyliques en $C_7$-$C_{13}$, et plus spécialement l'acide azélaïque sont des substances efficaces pour, en inhibant l'activité de la 5-$\alpha$ réductase, combattre les désordres signalés ci-dessus.

On a trouvé :

— d'une part, que les sels de zinc, notamment les sels de zinc solubles dans les milieu utilisé, ont également une action anti 5-$\alpha$ réductase ; cette activité qui peut d'ailleurs être de nature différente selon la concentration du sel de zinc, doit être mise en oeuvre dans la présente invention.

— d'autre part, que la vitamine $B_6$ potentialiserait l'action anti 5-$\alpha$ réductase des sels de zinc et qu'il convenait, par conséquent, que les compositions de la présente invention comportent également de la vitamine $B_6$.

La présente invention concerne donc les compositions cosmétiques et pharmaceutiques à application topique, caractérisées en ce qu'elles comportent :

— un acide dicarboxylique en $C_7$-$C_{13}$

— un sel de zinc

— de la vitamine $B_6$.

Par acide dicarboxylique en $C_7$-$C_{13}$, il faut entendre non seulement les acides eux-mêmes, mais leurs sels ou leurs dérivés solubles dans le milieu utilisé pour réaliser lesdites compositions. Parmi ces acides dicarboxyliques, l'acide préféré est l'acide azélaïque, qui peut s'utiliser soit sous forme d'acide, soit sous forme d'un sel de préférence le sel d'ammonium, le sel de sodium ou le sel de zinc soit sous la forme d'un sel d'éthanolamine.

On a montré que par utilisation de solutions très concentrées dudit acide dicarboxylique, il était possible d'obtenir une inhibition totale de l'activité de la 5-$\alpha$ réductase. Grâce à l'utilisation conjointe des sels de zinc et des vitamines $B_6$, la concentration de l'acide dicarboxylique dans les compositions selon l'invention est comprise entre 1 et 20% en poids.

Les sels de zinc utilisables sont les sels de zinc solubles dans les compositions selon l'invention. On utilisera, par exemple, pour les compositions aqueuses le sulfate de zinc ou le gluconate de zinc. Là encore, il n'est pas nécessaire que la concentration en sel de zinc soit, même compte tenu de la présente de la vitamine $B_6$, celle qui corresponde à une inhibition totale de la 5-$\alpha$ réductase. C'est ainsi que les compositions selon l'invention auront une concentration de 0,1 à 5% en poids de sel de zinc.

Enfin, la concentration de la vitamine $B_6$ doit être analogue à celle du sel de zinc, soit 0,1 à 5% en poids.

Les compositions selon l'invention se présentent sous forme de solutions, de gels, de crèmes ou de pommades et peuvent avantageusement comporter des additifs connus ayant pour fonction de faciliter l'acheminement du ou des principes actifs au niveau du follicule-sébacé.

Ces compositions sont, bien évidemment, appliquées sur la peau ou sur le cuir chevelu.

Les exemples non limitatifs suivants démontrent l'inhibition, in vitro, de l'activité de la 5-$\alpha$ réductase par les mélanges selon l'invention.

On a utilisé des solutions aqueuses contenant les divers produits selon l'invention à les concentrations convenables et on a mesuré l'activité d'une quantité constante de 5-$\alpha$ réductase mise en contact avec lesdites solutions.

Les résultats obtenus sont consignés dans le tableau unique ci-après ; dans une première colonne de ce tableau, on a indiqué la composition de la solution aqueuse utilisée et, dans la deuxième colonne, on a indiqué l'activité de la 5-$\alpha$ réductase après mise en contact pendant 60 min de la 5-$\alpha$ réductase avec ladite solution.

| Composition de la solution | Activité de la 5 - α réductase |
|---|---|
| Témoin (eau pure) | 100 % |
| Acide azélaïque à $1,5.10^{-2}$M | 1 % |
| Azélaate de sodium $1,5.10^{-2}$M | 35 % |
| Azélaate d'ammonium $1,5.10^{-2}$M | 1 % |
| Acide azélaïque de $0,2.10^{-3}$M | 83 % |
| Acide azélaïque $0,5.10^{-3}$M | 73 % |
| Sulfate de zinc $1,5.10^{-3}$M | 59 % |
| Sulfate de zinc $3.10^{-3}$M | 25 % |
| { Acide azélaïque $0,2.10^{-3}$M, Sulfate de zinc $1,5.10^{-3}$M } | 27 % |
| { Acide azélaïque $0,2.10^{-3}$M, Sulfate de zinc $3.10^{-3}$M } | 9 % |
| { Acide azélaïque $0,5.10^{-3}$M, Sulfate de zinc $1,5.10^{-3}$M } | 7,5 % |
| { Acide azélaïque $0,5.10^{-3}$M, Sulfate de zinc $3.10^{-3}$M } | 4,5 % |
| { Acide azélaïque $1.10^{-4}$M, Sulfate de zinc $0,5.10^{-3}$M, Vitamine $B_6$ 0,025 % } | 14 % |

Ces résultats démontrent l'effet, sur l'activité 5-α réductase, de l'acide azélaïque et l'effet potentialisateur, sur cette propriété de l'acide azélaïque, d'un sel de zinc et de la vitamine $B_6$.

**Revendications**

**Revendications pour les Etats contractants : BE CH DE FR GB IT LI LU NL**

1. Compositions cosmétiques et pharmaceutiques à application topique en vue de combattre les désordres liés à la 5-α réductase caractérisées en ce qu'elles contiennent :
— 1 à 20% en poids d'un acide dicarboxylique en $C_7$-$C_{13}$
— 0,1 à 5% en poids d'un sel soluble de zinc
— et de 0,1 à 5% en poids de vitamine $B_6$.
2. Compositions selon la revendication 1, caractérisées en ce que l'acide dicarboxylique est l'acide azélaïque.

**Revendications pour les Etats contractants : ES GR**

1. Compositions cosmétiques à application topique en vue de combattre les désordres liés à la 5-α réductase, caractérisées en ce qu'elles contiennent :
— 1 à 20% en poids d'un acide dicarboxylique en $C_7$-$C_{13}$ ;
— 0,1 à 5% en poids d'un sel soluble de zinc ;
— et de 0,1 à 5% en poids de vitamine $B_6$.
2. Compositions selon la revendication 1, caractérisées en ce que l'acide dicarboxylique est l'acide azélaïque.

3. Procédé de préparation de compositions cosmétiques et pharmaceutiques à application topique en vue de combattre les désordres liés à la 5-α réductase, caractérisées en ce qu'il comprend le mélange d'un excipient cosmétiquement ou pharmaceutiquement acceptable avec :
— 1 à 20% en poids d'un acide dicarboxylique en $C_7$-$C_{13}$ ;
— 0,1 à 5% en poids d'un sel soluble de zinc ;
— et 0,1 à 5% en poids de vitamine $B_6$.

4. Procédé de préparation selon la revendication 3, caractérisé en ce que l'acide dicarboxylique est l'acide azélaïque.

## Patentansprüche

### Patentansprüche für folgende Vertragsstaaten : BE CH DE FR GB IT LI LU NL

1. Kosmetische und pharmazeutische Zusammensetzungen zur topischen Anwendung zur Bekämpfung von mit 5-α-Reductase verbundenen Störungen, dadurch gekennzeichnet, daß sie enthalten :
— 1 bis 20 Gew.-% einer $C_{7-13}$-Dicarbonsäure,
— 0,1 bis 5 Gew.-% eines löslichen Zinksalzes und
— 0,1 bis 5 Gew.-% Vitamin B6.

2. Zusammensetzungen nach Anspruch 1, dadurch gekennzeichnet, daß die Dicarbonsäure Azelainsäure ist.

### Patentansprüche für folgende Vertragsstaaten : ES GR

1. Kosmetische Zusammensetzungen zur topischen Anwendung zur Bekämpfung von mit 5-α-Reductase verbundenen Störungen, dadurch gekennzeichnet, daß sie enthalten :
— 1 bis 20 Gew.-% einer $C_{7-13}$-Dicarbonsäure,
— 0,1 bis 5 Gew.-% eines löslichen Zinksalzes und
— 0,1 bis 5 Gew.-% Vitamin B6.

2. Zusammensetzungen nach Anspruch 1, dadurch gekennzeichnet, daß die Dicarbonsäure Azelainsäure ist.

3. Verfahren zur Herstellung von kosmetischen und pharmazeutischen Zusammensetzungen zur topischen Anwendung zur Bekämpfung von mit 5-α-Reductase verbundenen Störungen, gekennzeichnet durch Mischen eines kosmetisch oder pharmazeutisch akzeptablen Excipiens mit :
— 1 bis 20 Gew.-% einer $C_{7-13}$-Dicarbonsäure,
— 0,1 bis 5 Gew.-% eines löslichen Zinksalzes und
— 0,1 bis 5 Gew.-% Vitamin B6.

4. Herstellungsverfahren nach Anspruch 3, dadurch gekennzeichnet, daß die Dicarbonsäure Azelainsäure ist.

## Claims

### Claims for the following Contracting States : BE CH DE FR GB IT LI LU NL SE

1. Cosmetic and pharmaceutical compositions for topical application for the purpose of combating disorders linked to 5α-reductase, characterised in that they contain :
— 1 to 20% by weight of a $C_7$-$C_{13}$ dicarboxylic acid
— 0.1 to 5% by weight of a soluble zinc salt
— and from 0.1 to 5% by weight of vitamin B6.

2. Compositions according to Claim 1, characterised in that the dicarboxylic acid is azelaic acid.

### Claims for the following Contracting States : ES GR

1. Cosmetic compositions for topical application for the purpose of combating disorders linked to 5α-reductase, characterised in that they contain :
— 1 to 20% by weight of a $C_7$-$C_{13}$ dicarboxylic acid ;

— 0.1 to 5% by weight of a soluble zinc salt ;

— and from 0.1 to 5% by weight of vitamin B6.

2. Compositions according to Claim 1, characterised in that the dicarboxylic acid is azelaic acid.

3. Process for preparing cosmetic and pharmaceutical compositions for topical application for the purpose of combating disorders linked to 5α-reductase, characterised in that it comprises the mixing of a cosmetically or pharmaceutically acceptable excipient with :

— 1 to 20% by weight of a $C_7$-$C_{13}$ dicarboxylic acid ;

— 0.1 to 5% by weight of a soluble zinc salt ;

— and 0.1 to 5% by weight of vitamin B6.

4. Preparation process according to Claim 3, characterised in that the dicarboxylic acid is azelaic acid.